# EUROPÄISCHE PATENTSCHRIFT

(11) **EP 3 324 897 B1**
(45) Veröffentlichungstag und Bekanntmachung des Hinweises auf die Patenterteilung: **11.11.2020**
(21) Anmeldenummer: 16736175.7
(22) Anmeldetag: 07.07.2016
(51) Int. Cl.: A61F 2/50, A61F 2/76, A61F 2/80

(54) **VERFAHREN ZUM HERSTELLEN EINES PROTHESENSCHAFTES UND PROTHESENSCHAFT**
METHOD FOR MANUFACTURING A PROSTHETIC SOCKET AND PROSTHETIC SOCKET
PROCÉDURE DE FABRICATION D'UNE EMBOÎTURE DE PROTHÈSE ET EMBOÎTURE DE PROTHÈSE

(30) Priorität: 23.07.2015 DE 102015112028
(43) Veröffentlichungstag der Anmeldung: 30.05.2018
(60) Teilanmeldung: 20199317.7
(73) Patentinhaber: Ottobock SE & Co. KGaA, 37115 Duderstadt (DE)
(72) Erfinder: VAN VLIET, Johannis Willem, 3193 St. Aegyd am Neuwalde (AT); DIETL, Hans, 3003 Gablitz (AT)
(74) Vertreter: Gramm, Lins & Partner Patent- und Rechtsanwälte PartGmbB
(86) Internationale Anmeldenummer: PCT/EP2016/066169
(87) Internationale Veröffentlichungsnummer: WO 2017/012888

(56) Entgegenhaltungen:
- WO-A1-2013/142343
- US-A- 5 746 772
- US-A1- 2004 260 402
- US-B1- 6 358 453
- US-B1- 6 358 453
- HSU, L.H.: "The development of a rapid prototyping prosthetic socket coated with a resin layer for transtibial amputees", PROSTHETICS AND ORTHOTICS INTERNATIONAL, Bd. 34, Nr. 1, 1. März 2010 (2010-03-01), Seiten 37-45, XP055293801, DOI: 10.3109/03093640902911820
- NICHOLAS HERBERT ET AL: "A preliminary investigation into the development of 3-D printing of prosthetic sockets", JOURNAL OF REHABILITATION RESEARCH AND DEVELOPMENT, Bd. 42, Nr. 2, 1. Januar 2005 (2005-01-01) , Seite 141, XP055293669, US ISSN: 0748-7711, DOI: 10.1682/JRRD.2004.08.0134
- L. H. Hsu ET AL: "The development of a rapid prototyping prosthetic socket coated with a resin layer for transtibial amputees", PROSTHETICS AND ORTHOTICS INTERNATIONAL., vol. 34, no. 1, 1 March 2010 (2010-03-01), pages 37-45, XP055293801, GB ISSN: 0309-3646, DOI: 10.3109/03093640902911820

## Beschreibung

Die Erfindung betrifft ein Verfahren zum Herstellen eines Prothesenschaftes, bei dem ein 3D-Datensatz einer Außenkontur eines Stumpfes, an den der Prothesenschaft angelegt werden soll, erstellt und ein Basisschaft aus einem ersten Werkstoff in einem 3D-Druckverfahren unter Verwendung des 3D-Datensatzes erzeugt wird, wobei eine Innenkontur des Basisschafts der Außenkontur des Stumpfes entsprechen kann. Die Erfindung betrifft weiterhin einen Prothesenschaft zum Aufnehmen eines Stumpfes einer Extremität, mit einem Basisschaft mit einer Innenkontur, die einer Au-βenkontur des Stumpfes entspricht oder um eine Zugabe vergrößert ist, wobei der Basisschaft aus einem ersten Werkstoff besteht, der in einem 3D-Druckverfahren verarbeitbar ist.

Ein wesentlicher Aspekt bei der Herstellung von Prothesenschäften ist, dass die Innenkontur eines Prothesenschaftes für einen hohen Tragekomfort möglichst gut mit der Außenkontur eines Amputationsstumpfes übereinstimmt oder daran angepasst ist. Jeder Prothesenschaft wird somit idealerweise individuell für den Prothesenträger hergestellt, wobei die Form des Prothesenschaftes im Wesentlichen von der Form des Stumpfes sowie den zu erwartenden Belastungen durch Körpergewicht und Aktivität und der Tragesituation abhängt.

Für die Herstellung von Prothesenschäften sind unterschiedliche Herstellungsverfahren bekannt. Anfänglich wurden Prothesenschäfte als Holzschaft aus einem Stück Holz geschnitzt. Mit moderneren Fertigungsmaschinen und Werkstoffen konnten später Standardschäfte aus Metall oder Kunststoff hergestellt werden. Es wurden sogenannte Komposits entwickelt, die beispielsweise aus einer Kombination eines Prothesenschaftes aus Kunststoff und einem weichen Liner bestehen, der an einen Amputationsstumpf angelegt wird und der den Amputationsstumpf vor dem vergleichsweise harten Prothesenschaft schützt.

Es ist bekannt, Prothesenschäfte aus einem Laminierharz herzustellen. Dabei wird ein aushärtbares Harz und Fasermatten auf ein zuvor von einem Orthopädietechniker angefertigtes Gipsmodell aufgetragen und im Anschluss ausgehärtet. Der so entstandene Schaft ist verhältnismäßig steif und unnachgiebig. Der Schaft kann anschließend durch vergleichsweise teure Carbon-Elemente verstärkt werden, um eine adäquate Strukturfestigkeit zu erreichen. Aufgrund des steifen Prothesenschaftes ist ein zusätzlicher Prothesenliner und ein Verschlusssystem zum Verbinden des Prothesenliners mit dem Prothesenschaft vorzusehen.

Eine weitere Ausgestaltung eines Prothesenschaftes sieht einen weichen Innentrichter vor, der von einem harten Carbon-Rahmen gehalten wird. Der Amputationsstumpf, insbesondere ein Oberschenkelstumpf, ist dabei so in dem weichen Innentrichter eingebettet, dass der Prothesenschaft an seinem proximalen Rand flexibel ist und dadurch die Bewegungen der Extremität nur unwesentlich beeinträchtigt werden. Der Carbon-Rahmen weist darüber hinaus Anschlussmittel zu beispielsweise einem Prothesenkniegelenk auf. Auch bei dieser Art von Prothesenschaft ist zunächst ein Gipsmodell herzustellen, was die Herstellung aufgrund der zahlreichen Einzelschritte aufwendig sowie teuer macht, da auf die individuellen Erfordernisse eines Prothesenträgers Rücksicht genommen werden muss.

Zur Behebung der bekannten Nachteile werden seit jüngerer Zeit Prothesenschäfte auch im 3D-Druckverfahren hergestellt. Auf diese Weise ist es möglich, zahlreiche Herstellungsschritte automatisiert ablaufen zu lassen und somit den Aufwand und die Kosten zu reduzieren sowie gleichzeitig den individuellen Erfordernissen des Prothesenträgers in großem Maße gerecht zu werden.

Nachteilig an einem bislang im 3D-Druckverfahren hergestellten Prothesenschaft ist, dass die Strukturfestigkeit der verarbeitbaren Kunststoffe für einen dauerhaften Einsatz unter verschiedensten Umgebungsbedingungen ungeeignet ist.

Die Veröffentlichung "The development of a rapid prototyping prosthetic socket coated with a resin layer for transtibial amputees" von L.H. Hsu, in "Prosthetics and Orthotics International", Bd. 34, Nr. 1, vom 1. März 2010, XP055293801, stellt den nächstliegenden Stand der Technik dar und beschreibt einen Unterschenkelschaft, dessen innere Schicht mittels Rapid Prototyping, insbesondere Fused Deposition Modeling, gefertigt und mit einer äußeren Schicht aus ungesättigtem Polyesterharz verstärkt wird. Die Außenkontur des Stumpfes wird hierzu von einem portablen Handscanner vermessen, dessen Datenpunkte der Erstellung eines CAD-Modells dienen, die im Anschluss modifiziert werden,

Die Veröffentlichung "A preliminary investigation into the development of 3-D printing of prosthetic sockets" von Nicholas Herbert et al, in "Journal of Rehabilitation Research and Development", Bd. 42, Nr. 2, vom 1. Januar 2005, XP055293669, beschreibt den Einsatz von 3D-Druckverfahren zur Herstellung von Prothesenschäften. Insbesondere wird das Fused Deposition Modeling verwendet und der Prothesenschaft mit einem kohlefaserverstärkten Kunststoff umwickelt.

Die WO 2013/142343 A1 beschreibt ein Verfahren zur Herstellung einer mechanischen Schnittstelle zur Verbindung eines biologischen Körperteils mit einem tragbaren Gerät, z.B. einer Prothese oder Orthese. Es werden Beispiele eines 3D-Druckprozesses zur Herstellung eines Prototypen eines Prothesenschafts für einen Unterschenkelstumpf beschrieben.

Die US 6,358,453 B1 beschreibt ein Verfahren zur Herstellung eines Prothesenschafts mittels Rapid Prototyping, und die US 2004/0260402 A1 beschreibt ein Verfahren, bei dem ausgehend von einem 3D-Scan ein Prothesenschaft in einem digitalen Modell erstellt und auf Basis des Modells schichtweise aus einem Material aufgebaut wird.

Die US 5,746,772 A beschreibt einen Prothesenschaft mit einer eingearbeiteten Anschlussplatte.

Vor diesem Hintergrund ist Aufgabe der vorliegenden Erfindung, ein Verfahren zur Herstellung eines Prothesenschaftes bereit zu stellen, mit dem stabilere und über einen möglichst langen Tragezeitraum einsetzbare Prothesenschäfte hergestellt werden können.

Erfindungsgemäß wird diese Aufgabe durch ein Verfahren zum Herstellen eines Prothesenschaftes mit den Merkmalen des Hauptanspruchs sowie durch einen Prothesenschaft zum Aufnehmen eines Stumpfes einer Extremität mit den Merkmalen des nebengeordneten Anspruches gelöst. Vorteilhafte Ausgestaltungen und Weiterbildungen der Erfindung sind in den Unteransprüchen, der Beschreibung und den Figuren offenbart.

Das erfindungsgemäße Verfahren zum Herstellen eines Prothesenschaftes, bei dem ein 3D-Datensatz eine Außenkontur eines Stumpfes, an den ein Prothesenschaft angelegt werden soll, erstellt und ein Basisschaft aus einem ersten Werkstoff in einem 3D-Druckverfahren unter Verwendung des 3D-Datensatzes erzeugt wird, sieht vor, dass zumindest ein Stabilisierungselement aus einem zweiten Werkstoff auf den Basisschaft aufgebracht, z.B. auflaminiert, aufgeklebt, aufgeschweisst oder formschlüssig festgelegt wird. Durch das Aufbringen des Stabilisierungselements kann dem verhältnismäßig strukturschwachen, im 3D-Druckverfahren hergestellten Basisschaft die notwendige Strukturstärke verliehen werden, um dem Prothesenträger eine möglichst lange Tragedauer des Prothesenschaftes zu ermöglichen. Durch die Verwendung von unterschiedlichen Werkstoffen für den Basisschaft und das Stabilisierungselement können die individuellen, durch den Amputationsstumpf vorgegebenen, geometrischen Gegebenheiten sowie die notwendige Strukturfestigkeit in gleicher Weise berücksichtigt werden. Der Basisschaft kann dabei aus einem weicheren Werkstoff erzeugt werden und auf diese Weise der Tragekomfort des bereits im 3D-Druckverfahren präzise hergestellten und auf die Außenkontur des Stumpfes abgestimmten Prothesenschaftes weiter erhöht werden. Durch das Aufbringen, Auflaminieren, Aufschweißen, Aufkleben oder anderweitiges stoffschlüssiges, formschlüssiges oder kraftschlüssiges Festlegen zumindest eines Stabilisierungselements auf den Basisschaft wird eine Verbundstruktur gebildet, die die notwendige Strukturstärke für den täglichen Einsatz aufweist. Aufgrund des hochgradig automatisierten Herstellungsprozesses des Basisschafts im 3D-Druckverfahren sowie des technisch vergleichsweise einfachen nachträglichen Aufbringens oder Anbringes des Stabilisierungselements kann ein marktreifer und individueller Prothesenschaft mit einer hohen Geschwindigkeit und Fertigungsgüte hergestellt werden, die bislang in dieser Kombination nicht erreicht wurden.

Das Verfahren sieht vor, dass an dem Basisschaft ein Anker oder eine Anschlussplatte einlaminiert wird. An dem Basisschaft können Aufnahmen oder Positionierungshilfen für die an dem Basisschaft festzulegenden Komponenten ausgebildet werden, bevorzugt an der Außenseite des Basisschaftes, um an diesen Stellen die Komponenten anzuordnen und mit dem Basisschaft zu verbinden, beispielsweise durch Einlaminieren über Teile des Stabilisierungselementes. Dadurch entfällt vorteilhafterweise die mechanische Nachbearbeitung des Basisschafts, des Ankers und/oder der Anschlussplatte nahezu vollständig, da lediglich eine Grundpositionierung der Komponenten zueinander gewährleistet werden muss, nicht jedoch eine mechanische Verriegelung. Dadurch werden die Herstellungskosten und der zeitliche Aufwand weiter reduziert. Sofern die mechanischen Belastungen nicht zu hoch sind, kann der Anker oder die Anschlussplatte auch bei der Fertigung des Basisschaftes eingearbeitet und daran oder darin festgelegt werden.

Eine Weiterbildung des Verfahrens sieht vor, dass der 3D-Datensatz durch berührungslose Messverfahren, insbesondere optisches Vermessen mit einem 3D-Scanner, im Speziellen mit einem 3D-Laserscanner, durch Auswerten von Fotografien oder durch Ultraschalluntersuchungen, also insgesamt durch ein berührungsloses Erfassen der Außenkontur des Stumpfes, oder taktiles Vermessen des Stumpfes erstellt wird. Der Einsatz eines 3D-Laserscanners oder einer Digitalkamera zum Erstellen von auswertbaren Fotografien des Stumpfes hat für den Patienten den komfortablen Vorteil, dass der 3D-Datensatz der Außenkontur des Stumpfes berührungslos erstellt werden kann. Aufwendige Formabnahmen des Stumpfes, beispielsweise durch Gipsabformungen, entfallen dadurch gänzlich, wodurch eine hohe zeitliche Ersparnis erreicht wird. Als besonders vorteilhaft hat sich der Einsatz einer Digitalkamera herausgestellt, die mit einem Computer mit einer Auswertesoftware verbunden ist. Zur Erstellung des 3D-Datensatzes sind lediglich zwei Fotografien des Stumpfes, beispielsweise jeweils eine von frontaler und lateraler Seite, notwendig. Es können aber auch mehr als zwei Fotografien angefertigt und verwendet werden, beispielsweise Fotografien in allen Ebenen sowie in Richtung auf die Stumpfstirnfläche am distalen Ende des Stumpfes. Es kann auch eine umlaufende oder teilweise umlaufende Aufnahme des Stumpfes als Panoramafotografie erstellt und weiterverarbeitet werden. Eine Umlaufebene kann beispielsweise durch eine Stumpfmittellinie oder senkrecht dazu verlaufen. Die Daten der Fotografien werden über die Auswertesoftware in den 3D-Datensatz umgewandelt und können dem 3D-Druckverfahren zugrunde gelegt werden. Dadurch, dass lediglich eine Digitalkamera sowie ein Computer mit einer Auswertesoftware benötigt werden, stellt dies eine besonders günstige Möglichkeit dar, den 3D-Datensatz zu erstellen. Es können für den 3D-Datensatz auch 3D-Daten aus einer medizinischen Untersuchung beispielsweise in einem Magnetresonanz-Tomographen oder von Ultraschall-Untersuchungen verwendet werden. Dann kann auf die bereits vorhandenen Daten zugegriffen werden und eine zusätzliche Vermessung des Stumpfes gänzlich entfallen. Alternativ zu dem optischen, berührungslosen Verfahren kann der 3D-Datensatz auch mit einem taktilen Messverfahren ermittelt werden, beispielsweise unter Verwendung einer 3D-Koordinatenmessmaschine. Bei diesem Verfahren wird zunächst ein Referenzpunkt erstellt, von dem ausgehend die weiteren Messpunkte des Stumpfes präzise zueinander ermittelt werden. Der Referenzpunkt kann auch zwischen jedem weiteren Messpunkt neu ermittelt und ein Bezug zu dem alten Referenzpunkt hergestellt werden. Auf diese Weise können Abweichungen durch Bewegungen des Stumpfes während der Messung besser aus dem 3D-Datensatz herausgerechnet werden.

Bei einer Weiterbildung der Erfindung wird der erstellte 3D-Datensatz durch Eingeben und/oder Erfassen von zusätzlichen Stumpfdaten ergänzt. Der erstellte 3D-Datensatz der Außenkontur entspricht der später erzeugten Innenkontur des Basisschafts. Hierzu kann die Innenkontur exakt oder mit notwendigen Toleranzen und Materialzugaben gefertigt werden. Um die Schaftform und Schaftgestaltung optimal auf die individuellen Erfordernisse und Wünsche des Prothesenträgers abzustimmen, können beispielsweise das knöcherne ML-Maß und/oder das Perineum AP-Maß bestimmt und in den 3D-Datensatz eingespeist werden. Des Weiteren kann die Form des distalen Schaftendes als flache oder zunehmend spitze Form gestaltet werden oder der Prothesenschaft mit einem bestimmten Flexions- und Adduktionswinkel aufgebaut werden. Ebenfalls kann eine Ausnehmung für beispielsweise eine Druckpelotte im lateral-posterioren Bereich im 3D-Datensatz vorgesehen und später ausgedruckt werden. Darüber hinaus kann die Form der späteren Innenkontur und/oder Außenkontur des Prothesenschafts ausgewählt werden, um eine gewünschte Reibwirkung, haptische oder optische Wirkung zu erzielen. Im 3D-Druckverfahren kann dies beispielsweise durch einen Versatz von einzelnen oder mehreren Auftragsschichten zueinander und/oder durch mehrere Versätze innerhalb einer Auftragsschicht erzeugt werden, so dass eine in Umfangs- und/oder Längsrichtung geriffelte Oberfläche entsteht.

Bei einer Weiterbildung des Verfahrens wird der Basisschaft in einem 3D-Druckverfahren nach dem Prinzip des Polyjet-Modeling oder Fused Deposition Modeling mit Kunststoffen oder Kunstharzen erzeugt. Dadurch, dass eine Vielzahl von 3D-Druckern unterschiedlicher Hersteller bereits für den privaten Gebrauch auf dem Markt erwerbbar sind, sind die Kosten für 3D-Drucker, die nach dem Prinzip des Polyjet-Modeling oder Fused Deposition Modeling arbeiten, vergleichsweise gering. Je nach Größe des 3D-Druckers können prinzipiell auch mehrere individuelle Basisschäfte parallel in einem Druckauftrag hergestellt werden, was eine weitere Zeitersparnis sowie Kostenreduzierung mit sich bringt.

Bei einer weiteren Ausgestaltung des Verfahrens wird an dem Basisschaft zumindest eine Aufnahme für Anschlussmittel, beispielsweise für einen Anker oder eine Anschlussplatte, und/oder Hinterschneidungen oder Vorsprünge zur verbesserten Festlegung des Stabilisierungselementes im 3D-Druckverfahren erzeugt. Dazu werden die Daten für eine Aufnahme bereits in den 3D-Datensatz eingespeist, der Datensatz also insoweit modifiziert. Bei der Aufnahme kann es sich beispielsweise um Löcher, Anschläge oder Wände handeln, die ohne weitere mechanische Nachbearbeitung des Basisschafts bereits mit dem Basisschaft im 3D-Druckverfahren erzeugt werden. Der zeitliche Aufwand und die damit verbundenen Kosten für eine Nachbearbeitung des Basisschaftes entfallen damit gänzlich.

Vorteilhafterweise wird der Basisschaft in dem 3D-Druckverfahren über einen Umfang und/oder eine Längserstreckung mit einer inhomogenen Wandstärke versehen. Dazu werden in dem erstellten 3D-Datensatz der Außenkontur an unterschiedlichen Stellen verschiedene Zugaben oder Wandstärken zugeordnet, so dass sich eine ungleichmäßige Wandstärkenverteilung über den Umfang und/oder die Längserstreckung des Basisschaftes ergibt. Auf diese Weise können verschiedene Wandstärken gebildet und insbesondere so ausgewählt werden, dass stärker beanspruchte Bereiche mit einer höheren Wandstärke im Vergleich zu weniger beanspruchten Bereichen versehen werden. Dadurch kann der Basisschaft sowohl möglichst stabil als auch leicht ausgestaltet werden.

Vorzugsweise wird die Außenkontur automatisch mit der inhomogenen Wandstärke versehen. In Abhängigkeit von beispielsweise der Form und Lage des Stumpfes, des Gewichts und/oder der Knochenendform des Prothesenträgers kann der 3D-Datensatz automatisch auf die zu erwartenden Beanspruchungen angepasst und die benötigte Materialdicke automatisch an den entsprechenden Stellen zugegeben werden. Eine weitere Zeit- und Kostenreduzierung kann durch den weiter erhöhten Automatisierungsgrad erreicht werden. Ebenso ist es möglich und vorgesehen, dass anstatt der exakten Abformung der Außenkontur des Stumpfes zur Erstellung der Innenkontur des Basisschaftes der Außenkontur bei der Erstellung des Datensatzes für den Druckauftrag an ausgewählten Positionen eine Zugabe zugeordnet wird, so dass sich an bestimmten Stellen ein Freiraum für Volumenänderungen ergibt. Ebenso kann eine Verringerung des Volumens an bestimmten Stellen zu einer verbesserten Anlage des Basisschaftes an dem Stumpf führen, wenn beispielsweise bereichsweise der Umfang verringert wird.

In einer Ausführungsform des Verfahrens ist das zumindest eine Stabilisierungselement als ein Rahmen, ein Rahmenteil oder eine den Basisschaft umgebende Schicht ausgebildet und wird an der Außenseite des Basisschaftes angeordnet und festgelegt. Auf diese Weise kann die benötigte Strukturstabilität des Prothesenschaftes unter möglichst geringer Gewichtserhöhung und geringem Materialaufwand erreicht werden.

Vorzugsweise besteht das zumindest eine Stabilisierungselement aus Prepregs, die auf den Basisschaft aufgebracht, z.B. auflaminiert und anschließend unter Einfluss von Druck und Temperatur ausgehärtet werden. Prepregs können beispielsweise Textilfasern sein, die mit einem Reaktionsharz vorimprägniert wurden. Sie können beispielsweise als unidirektionale Schicht, als Gewebe oder als Gelege vorliegen und an die gewünschte Stelle oder die gewünschten Stellen auf den Basisschaft auflaminiert werden, um die Festigkeit des Basisschafts zu erhöhen. Die entsprechenden Stellen am Basisschaft können im Vorfeld mit einer Beanspruchungssimulation, beispielsweise mit Hilfe der Finite-Elemente-Methode und/oder einer Mehrkörpersimulation, unter Verwendung des 3D-Datensatzes ermittelt werden. Auf diese Weise können die Prepregs an einer optimalen Position und möglichst materialsparend eingesetzt werden.

Eine Innenkontur des Basisschafts kann der Außenkontur des Stumpfes entsprechen, so dass der Basisschaft und der Stumpf miteinander korrespondierend ausgebildet sind. Diese Ausgestaltung ist bei einer direkten Anlage des Basisschaftes auf der Hautoberfläche des Stumpfes vorteilhaft und insbesondere bei der Verwendung hautfreundlicher Materialien für den Basisschaft günstig. Alternativ kann die Außenkontur des Stumpfes erfasst und die sich unmittelbar ergebende Innenkontur mit einem Zuschlag versehen werden, so dass die Innenkontur formmäßig der Außenkontur des Stumpfes entspricht, jedoch im angelegten Zustand von der Hautoberfläche beabstandet wäre, um ausreichend Raum für ein Interface zwischen dem Stumpf und dem Basisschaft zu erhalten. Das Interface kann als Liner, beispielsweise aus einem Silikonwerkstoff oder einem anderen Polymer, ausgebildet sein, der Liner kann beschichtet sein, um ein Anhaften an der jeweiligen Oberfläche zu ermöglichen. Ebenso ist es möglich, dass das Interface ein Gewebe oder Gewirk, beispielsweise ein Abstandsgewirk aufweist oder als solches ausgebildet ist. Die Zugabe zu der Innenkontur kann der Dicke des Interfaces entsprechen oder geringfügig kleiner sein, um einen festen Sitz des Schaftes an dem Stumpf zu gewährleisten.

Der erfindungsgemäße Prothesenschaft zum Aufnehmen eines Stumpfes einer Extremität, mit einem Basisschaft mit einer Innenkontur, die einer Außenkontur des Stumpfes entspricht oder um eine Zugabe vergrößert ist, wobei der Basisschaft aus einem ersten Werkstoff besteht, der in einem 3D-Druckverfahren verarbeitbar ist, sieht zumindest ein auf den Basisschaft aufgebrachtes Stabilisierungselement aus einem zweiten Werkstoff vor, der eine höhere Strukturfestigkeit als der erste Werkstoff aufweist. Der erfindungsgemäße Prothesenschaft weist dadurch eine hohe Strukturfestigkeit auf und ist sehr präzise an den Stumpf des Prothesenträgers angepasst. Zusätzlich zu der äußerst wirtschaftlichen, wobei die Kosten für 3D-Druckverfahren in den kommenden Jahren noch weiter gesenkt werden können, und schnellen Herstellung, können die individuellen Erfordernisse des Prothesenträgers in hohem Maße berücksichtigt werden und somit ein hoher Qualitäts- und Komfortstandard erreicht werden. Das Stabilisierungselement ist vorteilhafter Weise auf der Au-βenseite des Basisschaftes befestigt, beispielsweise auflaminiert, aufgeklebt, aufgeschweisst, formschlüssig festgelegt oder anderweitig festgelegt, so dass die Innenkontur des Basisschaftes durch die Verstärkung nicht beeinflusst wird. Die Passform bleibt somit unabhängig von der Form und Beschaffenheit des Stabilisierungselementes. Der Prothesenschaft muss nicht zwingend passförmig zum Stumpf gefertigt werden. Es kann je nach Bedarf auch ein Abstand zwischen Innenkontur und Stumpf, beispielsweise für einen wärmenden Strumpf, einen Liner oder ein Abstandsgewirk vorgesehen werden. Es ist vorgesehen, dass der Basisschaft einen z.B. mittels des Stabilisierungselements eingearbeiteten, z.B. einlaminierten Anker oder eine eingearbeitete, z.B. einlaminierte Anschlussplatte aufweist. Es entfällt dadurch die Notwendigkeit der mechanischen Nachbearbeitung des Prothesenschafts und/oder des Ankers oder der Anschlussplatte. Die ansonsten durch beispielsweise ein Gewinde erzielte Verbindung wird durch ein einfaches Einbetten, Einlaminieren oder anderes Einarbeiten und Festlegen des Ankers oder der Anschlussplatte an oder in dem Basisschaft erreicht. Lediglich eine Grundpositionierung der Teile zueinander muss zuvor erfolgen, was eine unmittelbare Zeit- und Kostenersparnis mit sich bringt.

Bei einer Ausgestaltung des Prothesenschafts ist das Stabilisierungselement als Rahmen, Rahmenteil oder den Basisschaft umgebende Schicht ausgebildet. Das Stabilisierungselement kann beispielsweise als ein Ring oder mehrere voneinander in Längsrichtung beabstandete Ringe ausgebildet sein. Es können auch Versteifungsrippen zwischen zwei Ringen vorgesehen sein. Eine dünnwandige Schicht kann großflächig an dem Basisschaft ausgebildet sein, um eine gleichmäßige Strukturverstärkung zu erreichen. Mit allen Ausgestaltungen des Stabilisierungselements kann die benötigte Strukturstärke des Prothesenschaftes unter geringer Gewichtserhöhung und geringem Materialaufwand erreicht werden.

Bei einer bevorzugten Ausführungsform des Prothesenschaftes, weist der Basisschaft einen geschlossenen Querschnitt auf. Der Basisschaft ist dadurch formstabil und bedarf weniger Material des Stabilisierungselements zum Erreichen einer gewünschten Strukturfestigkeit des Prothesenschafts. Durch Verwendung von im Vergleich zum Stabilisierungselement günstigen Kunststoff oder Kunstharz für den Basisschaft können die Materialkosten weiter gesenkt werden.

Nachfolgend wird ein Ausführungsbeispiel der Erfindung anhand der Figuren näher erläutert. Es zeigen:
Fig. 1 - einen schematischen Herstellungsprozess für einen Prothesenschaft; sowie
Fig. 2 - einen Prothesenschaft mit montiertem Prothesenkniegelenk.

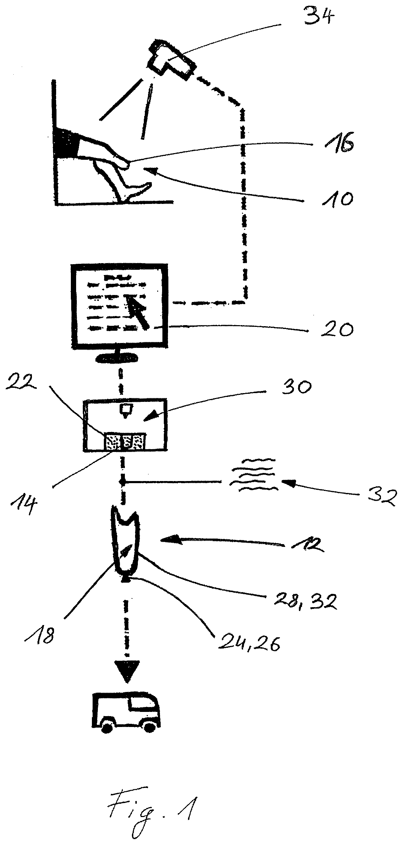

Gemäß dem in der Figur 1 beispielhaft dargestellten Herstellverfahren für einen Prothesenschaft werden zunächst mit Hilfe einer Digitalkamera 34 Fotografien der Au-βenkontur 16 eines Stumpfes 10 aufgenommen. Dabei können zwei Fotografien oder mehrere Fotografien aus unterschiedlichen Richtungen oder wie beschrieben ein zumindest teilweise umlaufendes Panoramabild gemacht werden. Aus den Fotografien wird mit Hilfe eines Computers und einer darauf befindlichen Auswertesoftware ein 3D-Datensatz 20 der Außenkontur 16 des Stumpfes 10 erzeugt. Der 3D-Datensatz 20, der bislang lediglich die Innenkontur 18 des späteren Basisschafts 14 und fertigen Prothesenschafts 12 darstellt, wird anschließend um weitere Daten ergänzt. Es hat sich gezeigt, dass mit Hilfe einer entsprechenden Software, z.B. Electronic-Test-Shaft-Software, TF oder TT Design Software oder CANFIT-Software der Firma Vorum Research Corporation, eine gewünschte Schaftform und Schaftgestaltung auf Grundlage des 3D-Datensatzes 20 erzeugt werden kann und weitere gewünschte Modifikationen schnell umsetzbar sind. Der modifizierte 3D-Datensatz 20 wird dann an einen 3D-Drucker 30 übergeben. Im 3D-Drucker 30, der in der dargestellten Ausführungsform nach dem Fused Deposition Modeling-Prinzip arbeitet, wird ein Kunststoff 22 oder Kunstharz 22 schichtweise aufgetragen und zu einem Basisschaft 14 schichtweise verschmolzen. Im nächsten Verfahrensschritt wird das Stabilisierungselement 32, das in der dargestellten Ausführungsform als Prepreg-Streifen oder - Matten vorliegt, auf den Basisschaft 14 auflaminiert. In der dargestellten Ausführungsform ist das Stabilisierungselement 32 als eine den Basisschaft 14 umgebende Schicht 28 ausgebildet. Des Weiteren ist an den Basisschaft 14 ein schematisch dargestellter Anker 24 oder eine Anschlussplatte 26 einlaminiert. Im Anschluss wird der zusammengestellte Basisschaft 14 in einer nicht dargestellten Kammer einem Druck und einer Temperatur ausgesetzt, die jeweils höher sind, als der Umgebungsdruck und die Umgebungstemperatur, so dass das Reaktionsharz der Prepregs aushärtet. Nachdem das Reaktionsharz ausgehärtet ist, ist der Prothesenschaft 12 fertig hergestellt und kann an den Prothesenträger ausgeliefert werden.

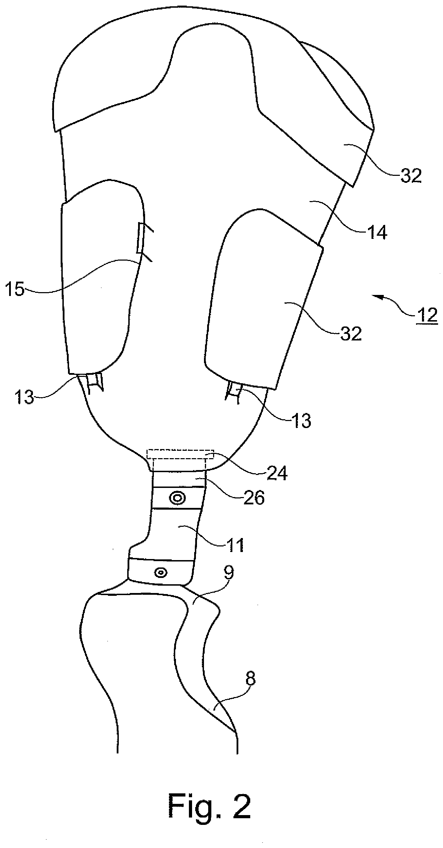

In der Figur 2 ist der fertiggestellte Prothesenschaft 12 im montierten Zustand gezeigt. Der Basisschaft 14 ist auf Grundlage des 3D-Datensatzes über den 3D-Drucker 30 erstellt worden. Die nicht dargestellte Innenkontur des Basisschaftes 14 ist dabei an die Außenkontur des Stumpfes angepasst, entspricht also entweder der Au-βenkontur exakt oder wurde geglättet oder mit einer Zugabe versehen, damit ein Interface oder ein Liner zwischen dem Stumpf und der Innenseite des Basisschaftes 14 angeordnet werden kann. Die Außenseite des Basisschaftes 14 kann eine im Wesentlichen glattwandige Oberfläche aufweisen, insofern sind unterschiedliche Wandstärken sowohl über den Umfang als auch über die Länge des Prothesenschaftes 12 beim Basisschaft 14 vorhanden. An der Außenseite des Basisschaftes 14 sind Vorsprünge 13 sowie Hinterschneidungen 15 eingearbeitet, die zur Positionierung und Fixierung von Stabilisierungselementen 32 dienen. In der Figur 2 ist am oberen Rand des Basisschaftes 14 ein umlaufendes erstes Stabilisierungselement 32 angeordnet und befestigt, beispielsweise in Gestalt von einem entsprechend geformten Prepreg oder mehrere Prepregs, die an dem semistabilen Basisschaft 14 angeordnet, vorfixiert und dann unter Aufbringung von Druck und Wärme ausgehärtet werden.

Unterhalb des ersten Stabilisierungselementes 32, der geschlossen umlaufend am oberen Rand des Prothesenschaftes 12 angeordnet ist, ist ein zweites Stabilisierungselement 32 angeordnet, dessen Unterkante auf den Vorsprüngen 13 aufsetzt und dessen eine Seitenkante in eine Hinterschneidung 15 greift. Das zweite Stabilisierungselement 32 weist eine spangenartige Kontur auf, das heißt, dass das zweite Stabilisierungselement 32 den Umfang des Basisschaftes 14 nur teilweise umgreift. Dadurch wird eine zwar erhöhte Stabilität des Basisschaftes 14 im Bereich des zweiten Stabilisierungselementes 32 bewirkt, aufgrund des offenen Umfangsquerschnittes ist es jedoch möglich, eine gewisse Elastizität bereitzustellen, auch wenn das Material der Stabilisierungselemente 32 eine wesentlich höhere Festigkeit gegenüber einer Verformung aufweist als das Material des Basisschaftes.

Weiterhin ist in der Figur 2 dargestellt, dass die Anschlussplatte 26 sowie der Anker 24 in dem Basisschaft 14 eingesetzt und befestigt oder einlaminiert sind. Im Bereich der Anschlussplatte 26 und des Ankers 24 ist eine Materialaufdickung im Basisschaft 14 an der Außenseite vorgesehen, um eine verbesserte Stabilität und ausreichend Raum für die Aufnahme des Komponenten zu haben. Die Materialaufdickung kann auch über das Stabilisierungselement und/oder die Beschichtung auf der Außenseite des Basisschaftes 14 erfolgen. Die Befestigung der Anschlussplatte 26 und des Ankers 24 kann neben der Einbettung in das Material des Basisschaftes und/oder des Stabilisierungselementes auch herkömmlich mechanisch erfolgen, beispielsweise durch Verschrauben und/oder Verkleben. Im Bereich der Befestigungsstelle der Anschlussplatte 26 und des Ankers 24 können entsprechend gestaltete Aufnahmebereiche, Ausrichteinrichtungen und/oder Anschläge ausgebildet sein, um die Positionierung der Komponenten zu erleichtern. An der Anschlussplatte 26 ist ein Anschlussadapter 11 zum Längenausgleich vorgesehen, der wiederum mit dem nur angedeuteten Prothesenkniegelenk 9 gekoppelt ist, so dass der Prothesenschaft 12 zur Aufnahme eines Oberschenkelstumpfes mit einem Unterschenkelteil 3 schwenkbar verbunden ist.

Der Basisschaft 14 kann zusätzlich mit einer nicht dargestellten Beschichtung versehen sein, die die komplette Außenseite des Basisschaftes 14 abdeckt. Die Beschichtung kann in einem Tauchverfahren oder einem anderen Auftragverfahren aufgebracht werden. Alternativ kann die Beschichtung auch nur teilweise an der Außenseite des Basisschaftes 14 aufgebracht sein, ebenso kann die Beschichtung auf der Innenseite des Basisschaftes 14 vollflächig oder nur teilweise aufgebracht sein. Über die Beschichtung können die Funktionalität und die Haptik sowie die Oberflächengestalt des Basisschaftes 14 eingestellt, insbesondere an den jeweiligen Nutzungszweck angepasst werden. Die Beschichtung und/oder die Stabilisierungselemente können nach dem Aufbringen auf den Basisschaft nachbehandelt werden, beispielsweise einer Wärmebehandlung unterzogen oder bestrahlt werden, um die gewünschten Festigkeitseigenschaften zu erzielen. So kann auf der Innenseite eine hautfreundliche Beschichtung aufgebracht werden, während auf der Außenseite des Basisschaftes 14 eine schmutzabweisende oder stabilisierende Beschichtung aufgebracht sein kann.

### Bezugszeichenliste

- 8: Unterschenkelteil
- 9: Prothesenkniegelenk
- 10: Stumpf
- 11: Adapter
- 12: Prothesenschaft
- 13: Vorsprung
- 14: Basisschaft
- 15: Hinterschneidung
- 16: Außenkontur
- 18: Innenkontur
- 20: 3D-Datensatz
- 22: Kunststoff / Kunstharz
- 24: Anker
- 26: Anschlussplatte
- 28: Schicht
- 30: 3D-Drucker
- 32: Stabilisierungselement
- 34: Digitalkamera

## Patentansprüche

1. Verfahren zum Herstellen eines Prothesenschaftes (12), bei dem ein 3D-Datensatz (20) einer Außenkontur (16) eines Stumpfes (10), an den ein Prothesenschaft (12) angelegt werden soll, erstellt und ein Basisschaft (14) aus einem ersten Werkstoff in einem 3D-Druckverfahren unter Verwendung des 3D-Datensatzes (20) erzeugt wird, wobei zumindest ein Stabilisierungselement (32) aus einem zweiten Werkstoff auf den Basisschaft (14) aufgebracht wird, **dadurch gekennzeichnet, dass** an den Basisschaft (14) ein Anker (24) oder eine Anschlussplatte (26) eingearbeitet wird.

2. Verfahren nach Anspruch 1, **dadurch gekennzeichnet, dass** der 3D-Datensatz (20) durch berührungslose Messverfahren oder durch taktiles Vermessen des Stumpfes (10) erstellt wird.

3. Verfahren nach Anspruch 1 oder 2, **dadurch gekennzeichnet, dass** der 3D-Datensatz (20) durch Eingeben und/oder Erfassen von zusätzlichen Stumpfdaten ergänzt wird.

4. Verfahren nach einem der voranstehenden Ansprüche, **dadurch gekennzeichnet, dass** das 3D-Druckverfahren nach dem Prinzip des Polyjet Modeling oder Fused Deposition Modeling mit Kunststoffen (22) und/oder Kunstharzen (22) erfolgt.

5. Verfahren nach einem der voranstehenden Ansprüche, **dadurch gekennzeichnet, dass** an dem Basisschaft (14) eine Aufnahme für Anschlussmittel und/oder Hinterschneidungen (15) oder Vorsprünge (13) für die Festlegung des Stabilisierungselementes (32) im 3D-Druckverfahren erzeugt werden.

6. Verfahren nach einem der voranstehenden Ansprüche, **dadurch gekennzeichnet, dass** der Basisschaft (14) über seinen Umfang und/oder seine Längserstreckung mit einer inhomogenen Wandstärke gedruckt wird.

7. Verfahren nach Anspruch 6, **dadurch gekennzeichnet, dass** in dem 3D-Datensatz die Außenkontur (16) automatisch mit der inhomogenen Wandstärke beaufschlagt wird.

8. Verfahren nach einem der voranstehenden Ansprüche, **dadurch gekennzeichnet, dass** das zumindest eine Stabilisierungselement (32) als ein Rahmen, ein Rahmenteil oder eine den Basisschaft (14) umgebende Schicht (28) ausgebildet ist und an dem Basisschaft (14) angeordnet.

9. Verfahren nach einem der voranstehenden Ansprüche, **dadurch gekennzeichnet, dass** das zumindest eine Stabilisierungselement (32) aus Prepregs besteht, die auf den Basisschaft (14) aufgebracht und unter Einfluss von Druck und Temperatur ausgehärtet werden.

10. Verfahren nach einem der voranstehenden Ansprüche, **dadurch gekennzeichnet, dass** eine Innenkontur (18) des Basisschafts (14) der Außenkontur (16) des Stumpfes (10) entspricht oder um die Dicke eines Interfaces zwischen Innenkontur (18) und Außenkontur (16) vergrößert wird.

11. Prothesenschaft (12) zum Aufnehmen eines Stumpfes (10) einer Extremität, mit einem Basisschaft (14) mit einer Innenkontur (18), die einer Außenkontur (16) des Stumpfes (10) entspricht oder um eine Zugabe vergrößert ist, der Basisschaft (14) besteht aus einem ersten Werkstoff, der in einem 3D-Druckverfahren verarbeitbar ist, und zumindest einem auf den Basisschaft (14) aufgebrachten Stabilisierungselement (32) aus einem zweiten Werkstoff, der eine höhere Strukturfestigkeit als der erste Werkstoff aufweist, **dadurch gekennzeichnet, dass** der Basisschaft (14) einen eingearbeiteten Anker (24) oder eine eingearbeitete Anschlussplatte (26) aufweist.

12. Prothesenschaft (12) nach Anspruch 11, **dadurch gekennzeichnet, dass** das Stabilisierungselement (32) als Rahmen, Rahmenteil oder den Basisschaft (14) umgebende Schicht (28) ausgebildet ist.

13. Prothesenschaft (12) nach einem der Ansprüche 11 oder 12, **dadurch gekennzeichnet, dass** der Basisschaft (14) einen geschlossenen Querschnitt aufweist.

## Claims

1. A method for producing a prosthesis socket (12), in which a 3D data set (20) of an outer contour (16) of a stump (10), to which a prosthesis socket (12) should be applied, is created and a base socket (14) is produced from a first substance in a 3D printing method using the 3D data set (20), **wherein** at least one stabilization element (32) that is made of a second substance is applied to the base socket (14), **characterized in that** an anchor (24) or a connecting plate (26) is worked into the base socket (14).

2. The method as claimed in claim 1, **characterized in that** the 3D data set (20) is created by a contactless measurement method or by a tactile measurement of the stump (10).

3. The method as claimed in claim 1 or 2, **characterized in that** the 3D data set (20) is complemented by entering and/or capturing additional stump data.

4. The method as claimed in any one of the preceding claims, **characterized in that** the 3D printing method is carried out according to the principle of polyjet modeling or fused deposition modeling with polymers (22) and/or synthetic resins (22).

5. The method as claimed in any one of the preceding claims, **characterized in that** a receptacle for connection means and/or undercuts (15) or projections (13) for fixing the stabilization element (32) are produced on the base socket (14) during the 3D printing method.

6. The method as claimed in any one of the preceding claims, **characterized in that** the base socket (14) is printed with an inhomogeneous wall thickness over its circumference and/or its longitudinal extent.

7. The method as claimed in claim 6, **characterized in that**, in the 3D data set, the outer contour (16) automatically has the inhomogeneous wall thickness applied thereto.

8. The method as claimed in any one of the preceding claims, **characterized in that** the at least one stabilization element (32) is embodied as a frame, a frame part or a layer (28) that surrounds the base socket (14), and arranged at the base socket (14).

9. The method as claimed in any one of the preceding claims, **characterized in that** the at least one stabilization element (32) consists of pre-pregs that are applied to the base socket (14) and cured under the influence of pressure and temperature.

10. The method as claimed in any one of the preceding claims, **characterized in that** an inner contour (18) of the base socket (14) corresponds to the outer contour (16) of the stump (10) or said inner contour is increased in size by the thickness of an interface between the inner contour (18) and outer contour (16).

11. A prosthesis socket (12) for receiving a stump (10) of an extremity, having a base socket (14) with an inner contour (18) which corresponds to an outer contour (16) of the stump (10) or which is increased in size by an allowance, the base socket (14) consisting of a first substance which is processable in a 3D printing method, and at least one stabilization element (32) applied to the base socket (14), said stabilization element being made of a second substance which has a higher structural strength than the first substance, **characterized in that** the base socket (14) has a built-in anchor (24) or a built-in connecting plate (26).

12. The prosthesis socket (12) as claimed in claim 11, **characterized in that** the stabilization element (32) is embodied as a frame, a frame part or a layer (28) that surrounds the base socket (14).

13. The prosthesis socket (12) as claimed in any one of claims 12 to 14, **characterized in that** the base socket (14) has a closed cross section.

## Revendications

1. Procédé de réalisation d'une emboîture de prothèse (12), dans lequel on établit un jeu de données 3D (20) d'un contour extérieur (16) d'un moignon (10) sur lequel une emboîture de prothèse (12) doit être appliquée, et on réalise une emboîture de base (14) en un premier matériau dans un procédé d'impression 3D en utilisant le jeu de données 3D (20), au moins un élément de stabilisation (32) en un deuxième matériau étant placé sur l'emboîture de base (14),
**caractérisé en ce que**
un ancrage (24) ou une plaque de raccordement (26) est intégré(e) sur l'emboîture de base (14).

2. Procédé selon la revendication 1,
**caractérisé en ce que** le jeu de données 3D (20) est établi par des méthodes de mesure sans contact ou par mesure tactile du moignon (10).

3. Procédé selon la revendication 1 ou 2,
**caractérisé en ce que** le jeu de données 3D (20) est complété par la saisie et/ou l'enregistrement de données supplémentaires sur le moignon.

4. Procédé selon l'une des revendications précédentes,
**caractérisé en ce que** le procédé d'impression 3D est réalisé selon le principe de la modélisation par polyjet (Polyjet Modeling) ou de la modélisation par déposition de matériaux en fusion (Fused Deposition Modeling) avec des matières plastiques (22) et/ou des résines synthétiques (22).

5. Procédé selon l'une des revendications précédentes,
**caractérisé en ce qu'**un logement pour des moyens de raccordement et/ou des contre-dépouilles (15) ou des saillies (13) pour l'immobilisation de l'élément de stabilisation (32) sont réalisés sur l'emboîture de base (14) dans le procédé d'impression 3D.

6. Procédé selon l'une des revendications précédentes,
**caractérisé en ce que** l'emboîture de base (14) est imprimée sur sa circonférence et/ou son extension longitudinale avec une épaisseur de paroi non homogène.

7. Procédé selon la revendication 6,
**caractérisé en ce que** dans le jeu de données 3D, le contour extérieur (16) est automatiquement pourvu de l'épaisseur de paroi non homogène.

8. Procédé selon l'une des revendications précédentes,
**caractérisé en ce que** ledit au moins un élément de stabilisation (32) est réalisé sous la forme d'un cadre, d'une partie de cadre ou d'une couche (28) entourant l'emboîture de base (14), et est disposé sur l'emboîture de base (14).

9. Procédé selon l'une des revendications précédentes,
**caractérisé en ce que** ledit au moins un élément de stabilisation (32) est constitué de préimprégnés qui sont appliqués sur l'emboîture de base (14) et qui sont durcis sous l'influence de pression et de température.

10. Procédé selon l'une des revendications précédentes,
**caractérisé en ce qu'**un contour intérieur (18) de l'emboîture de base (14) correspond au contour extérieur (16) du moignon (10) ou est augmenté de l'épaisseur d'une interface entre le contour intérieur (18) et le contour extérieur (16).

11. Emboîture de prothèse (12) destinée à recevoir un moignon (10) d'une extrémité, comprenant une emboîture de base (14) ayant un contour intérieur (18) qui correspond à un contour extérieur (16) du moignon (10) ou qui est agrandi par un ajout, l'emboîture de base (14) étant constituée d'un premier matériau qui peut être traité par un procédé d'impression en 3D, et au moins un élément de stabilisation (32) en un deuxième matériau qui est appliqué sur l'emboîture de base (14) et qui présente une résistance structurelle plus élevée que le premier matériau,
**caractérisée en ce que**
l'emboîture de base (14) comprend un ancrage (24) intégré ou une plaque de raccordement (26) intégrée.

12. Emboîture de prothèse (12) selon la revendication 11,
**caractérisée en ce que** l'élément de stabilisation (32) est réalisé sous la forme d'un cadre, d'une partie de cadre ou d'une couche (28) entourant l'emboîture de base (14).

13. Emboîture de prothèse (12) selon l'une des revendications 11 ou 12, **caractérisée en ce que** l'emboîture de base (14) présente une section transversale fermée.
